# EUROPEAN PATENT APPLICATION

(11) **EP 4 792 867 A1**
(43) Date of publication of application: **19.08.2026**
(21) Application number: 24893473.9
(22) Date of filing: 20.11.2024
(51) Int. Cl.: A61M 27/00, A61M 25/00

(54) **SHUNT FOR TREATING HYDROCEPHALUS AND SYSTEM THEREOF**

(30) Priority: 24.11.2023 CN 202311579340
(71) Applicant: Ton-Bridge Medical Technology Co., Ltd, Zhuhai, Guangdong 519085 (CN)
(72) Inventor: LI, Zheng, Zhuhai, Guangdong 519085 (CN); LIU, Zhihua, Zhuhai, Guangdong 519085 (CN); LIU, Xiangcheng, Zhuhai, Guangdong 519085 (CN); ZHANG, Mingqiang, Zhuhai, Guangdong 519085 (CN)
(74) Representative: Patent 42
(86) International application number: PCT/CN2024/133265
(87) International publication number: WO 2025/108317

(57) **Abstract**

Provided are a shunt and system for treating hydrocephalus. The shunt includes a distal portion provided with a cerebrospinal fluid inlet, a proximal portion provided with a cerebrospinal fluid outlet, and a tube body provided with a flow lumen. The distal portion is provided with a limiting portion in contact with a dura mater. A maximum radial size of the limiting portion in a delivery state is greater than both a minimum radial size of a puncture opening and a minimum radial size of the tube body. The system includes the shunt and a delivery system that is configured to deliver the shunt to designated positions in a venous system and a ventricular system. During delivery, the shunt is placed inside the delivery system. The distal portion of the shunt is configured to dilate the puncture opening in a dura mater and push the limiting portion into a cistern.

## Description

### TECHNICAL FIELD

The present disclosure relates to the field of medical device technologies, and in particular, to a shunt and system for treating hydrocephalus.

### BACKGROUND

Hydrocephalus is one of the most prevalent and significant neurosurgical disorders affecting both children and adults. The term hydrocephalus, meaning "water on the brain," refers to an abnormal accumulation of cerebrospinal fluid (CSF) within the brain. The excessive intracranial pressure caused by hydrocephalus can lead to numerous pronounced symptoms, ranging from headaches to neurological dysfunction, coma, and death. CSF is a clear physiological fluid in which the entire nervous system (including the brain and spinal cord) is bathed. CSF is produced by cells of the choroid plexus located within the cerebral ventricles. In a normal patient, the CSF produced in the choroid plexus is reabsorbed by cells within the arachnoid granulations. The arachnoid granulations span the surface of the brain's intracranial venous drainage system and reabsorb CSF present in the subarachnoid space into the venous system. Approximately 450 mL to 500 mL of CSF is produced and reabsorbed daily, enabling a steady-state volume and pressure of approximately 8 to 16 cm H2O within the intracranial ventricles. This reabsorption pathway has been termed the "third circulation" because of its importance to central nervous system homeostasis.

Hydrocephalus most often occurs as a result of impaired CSF reabsorption, and occasionally due to oversecretion of the CSF. The condition with impaired reabsorption is called communicating hydrocephalus. Hydrocephalus can also arise from a partial or complete blockage of one of the CSF pathways, including the cerebral aqueduct (aqueduct of Sylvius), leading to a condition known as obstructive hydrocephalus. Normal pressure hydrocephalus (NPH) is a form of communicating hydrocephalus. Unlike other forms of communicating hydrocephalus, patients with NPH may exhibit little or no increase in intracranial pressure. It is believed that in NPH patients, the CSF-filled ventricles within the brain enlarge to accommodate the increased CSF volume in the subarachnoid space.

In recent years, a percutaneous/endovascular interventional treatment method for CSF shunting has been proposed, in which a shunt is deployed in the patient's inferior petrosal sinus (IPS) and the cerebellopontine angle cistern (CP angle cistern). Specifically, the distal portion of the shunt is introduced via the IPS and secured within the patient's CP angle cistern, which includes CSF; the proximal portion of the shunt is secured in or near the patient's jugular vein (JV); CSF flows from the CP angle cistern into the JV through the flow channel of the shunt, thereby maintaining a normal pressure differential between the subarachnoid space and venous system of the patient. However, shunt systems suitable for this treatment method are still relatively few, and have problems including high costs, a complex structure, and the like.

Therefore, there is an urgent need for a shunt for treating hydrocephalus.

### SUMMARY

In view of this, the present disclosure proposes a shunt and system for treating hydrocephalus, to help simplify a structure of the shunt and optimize the system.

The technical solution of the present disclosure is implemented as follows: A shunt for treating hydrocephalus, configured to be deployed in a ventricular system and a venous system of a patient to drain cerebrospinal fluid from the ventricular system of the patient into the venous system of the patient, includes: a distal portion provided with a cerebrospinal fluid inlet, a proximal portion provided with a cerebrospinal fluid outlet, and a tube body provided with a flow lumen, where a dura mater between the ventricular system and the venous system is provided with a puncture opening, the distal portion is configured to be disposed in the ventricular system, the proximal portion is configured to be disposed in the venous system, the distal portion is connected to the proximal portion through the tube body, the cerebrospinal fluid inlet communicates with the cerebrospinal fluid outlet through the flow lumen, the distal portion is provided with a limiting portion configured to be in contact with the dura mater, and a maximum radial size of the limiting portion in a delivery state is greater than a minimum radial size of the puncture opening and a minimum radial size of the tube body.

Based on the above technical solution, preferably, a maximum radial size of a distal end of the tube body is greater than the minimum radial size of the puncture opening.

Based on the above technical solution, preferably, the distal portion is provided with an expanding portion connected to the limiting portion, the expanding portion is farther from the proximal portion than the limiting portion, and a radial size of the expanding portion gradually increases in a direction from the distal portion to the proximal portion.

Based on the above technical solution, preferably, a minimum radial size of the expanding portion is less than or equal to a maximum radial size of the puncture opening.

Based on the above technical solution, preferably, the limiting portion is in a shape of a truncated cone, an ellipsoid, a prism, a cylinder, or a cone; and/or
a distal end of the tube body close to the distal portion is circular or flat; and/or
a central axis of the limiting portion coincides with or does not coincide with a central axis of the tube body.

Based on the above technical solution, preferably, the limiting portion is made of an elastic material; or the limiting portion includes an elastic structure, and the elastic structure is located at a distal end of the limiting portion.

Based on the above technical solution, preferably, a difference between the maximum radial size of the limiting portion and the minimum radial size of the puncture opening is a maximum limiting size, a difference between a minimum radial size of the limiting portion and a maximum radial size of the puncture opening is a minimum limiting size, and the maximum limiting size and the minimum limiting size range from 0 mm to 3 mm.

Based on the above technical solution, preferably, the tube body is provided with a stepped surface close to the distal portion, the stepped surface is configured to form limitation with a side of the dura mater away from the ventricular system, and a distal tube segment having a minimum radial size smaller than a maximum radial size of the tube body is provided between the stepped surface and the limiting portion.

Based on the above technical solution, preferably, a sealant is provided between the tube body and the puncture opening.

Based on the above technical solution, preferably, the shunt further includes a flow direction control member, where the flow direction control member is configured to allow the cerebrospinal fluid to flow only from the ventricular system into the venous system through the flow lumen, and the flow direction control member is disposed in the flow lumen, or on the proximal portion, or on the distal portion, or a combination thereof.

Based on the above technical solution, preferably, the flow direction control member is a one-way valve, a unidirectional flow channel, a unidirectional flow surface, or a spring valve.

Based on the above technical solution, preferably, the one-way valve is a duckbill valve, a slit valve, a bicuspid valve, or a monocuspid valve; the unidirectional flow channel is a Tesla valve; and the unidirectional flow surface is disposed as a unidirectional flow microstructure, a hydrophilic-hydrophobic alternating structure, or a unidirectional damping-increasing surface structure.

Based on the above technical solution, preferably, a semipermeable membrane is provided in the distal portion, the proximal portion, or the tube body, and the semipermeable membrane is configured to restrict flow, diffusion, or exchange of some components between the ventricular system and the venous system; and/or
a polymer liner and/or an antithrombogenic coating is further included, where the polymer liner and/or the antithrombogenic coating is disposed on the tube body, the proximal portion, and the distal portion; and/or
the proximal portion is provided with a shielding protection device, and the shielding protection device is a stent, a balloon, or a membrane; and/or
at least one radiopaque marker is set on the shunt.

Another technical solution of the present disclosure is a system for treating hydrocephalus includes the shunt and a delivery system, where the delivery system is configured to deliver the shunt to designated positions in the venous system and the ventricular system, and during delivery, the shunt is placed inside the delivery system.

Based on the above technical solution, preferably, the delivery system includes a delivery catheter, a shunt pusher, a puncture member, and a guide member, where during delivery, the shunt, the shunt pusher, the puncture member, and the guide member are all located inside the delivery catheter, and the puncture member is disposed outside the shunt, inside the shunt, or on the distal portion.

Based on the above technical solution, preferably, a distal end of the puncture member is provided with a puncture tip having a cutting edge, the puncture tip is configured to puncture a dura mater to form a puncture opening in the dura mater, and the puncture tip is a circular or flat thin blade.

Based on the above technical solution, preferably, a puncture protective sheath is provided outside the puncture tip.

Based on the above technical solution, preferably, the distal end of the puncture member is provided with a radiopaque marker.

Based on the above technical solution, preferably, a distal anchoring member is connected to a distal end of the guide member, and/or a proximal deflection member is connected to a proximal end of the guide member.

Based on the above technical solution, preferably, the delivery system further includes a dilation member configured to dilate the puncture opening; and/or
the delivery system includes at least one radiopaque marker; and/or
the delivery system is connected to an operation handle, and the operation handle is configured to operate the delivery system to deliver the shunt.

Compared with the conventional technology, the shunt for treating hydrocephalus and the system thereof according to the present disclosure have the following beneficial effects:
(1) In the present disclosure, the distal portion of the shunt is configured to dilate the puncture opening in the dura mater and squeeze the limiting portion, which is larger than the puncture opening in size, into the cistern. By utilizing the elastic recovery capability of the dura mater, the limiting portion forms a snap-fit anchor with the dura mater. The limiting portion does not expand when deployed in the ventricular system, that is, it does not expand before or after entering the cistern. The limiting portion provides limitation by virtue of its own size, resulting in a simple structure and simple operation, and enabling simplification of the delivery process and the delivery system. The maximum radial size of the limiting portion is greater than the size of the puncture opening both in the delivery state before entering the ventricle and in the deployed state after entering the ventricle. As used herein, "the limiting portion does not expand" means that the limiting portion does not undergo significant deformation that would affect its limiting function, and does not cover minor deformation of the material of the limiting portion itself caused by factors including different temperatures, and the like. The puncture opening referred to herein is an opening formed in the dura mater by puncturing the dura mater with a puncture member. Experimental tests of the present disclosure have shown that because the dura mater has a certain elastic deformation capability, the dura mater elastically deforms during puncture and elastically recovers after puncture, that is, the puncture opening has a certain deformation recovery margin. Therefore, the size of the puncture opening formed in the dura mater after puncture is smaller than the size of the puncture member used, and the limiting portion of the shunt is larger than the size of the puncture opening, allowing the limiting portion to form a snap-fit with the dura mater. Even if the dura mater tears beyond elastic deformation during puncture with the puncture member, because the dura mater is a soft membrane material, a shunt with a larger tube body size can be used to dilate the puncture opening, thereby further closing the torn site. Specifically, using a puncture needle with an outer diameter of 0.9 mm and a willow-leaf blade, the diameter of the puncture opening is only 0.45 mm; and using a puncture needle with an outer diameter of 0.9 mm and a half-pointed tip, the diameter of the puncture opening is less than 0.6 mm. Therefore, using a limiting portion larger than the puncture needle diameter and a tube body slightly larger than the puncture opening enables fixation and sealing of the shunt. In addition, calculations show that the force on the shunt in the venous system is very small, thus requiring low anchoring force and consequently imposing low requirements on the size of the distal limiting portion.
(2) The radial size of the distal end of the tube body of the present disclosure is greater than that of the puncture opening. Due to the elastic deformation capability of the dura mater, an interference fit is achieved between the tube body and the puncture opening, thereby achieving a sealing effect.
(3) When the puncture member of the present disclosure is configured as a flat thin blade, the flat thin blade puncture member can fully utilize the space of the flow lumen of the shunt while maximizing the occupied size, that is the resulting puncture opening is smaller, which is more favorable for the limiting portion to achieve limitation. Meanwhile, because the flat thin blade puncture member has the smallest size, the resulting puncture opening is smaller, which is more favorable for achieving sealing between the tube body and the puncture opening. The puncture protective sheath, while serving as a shunt pusher, provides protection for the shunt liner, preventing the puncture needle from cutting and damaging the inner wall of the shunt and the duckbill valve. The puncture member may also be configured as a circular shape. A circular puncture member creates a larger puncture opening than a flat thin blade puncture member, requiring selection of a larger limiting portion and tube body for limitation and sealing with the puncture opening.

### BRIEF DESCRIPTION OF THE DRAWINGS

To describe the technical solutions in the embodiments of the present disclosure or in the prior art more clearly, the following briefly describes the accompanying drawings required for describing the embodiments or the prior art. Apparently, the accompanying drawings in the following description show merely some embodiments of the present disclosure, and a person of ordinary skill in the art may still derive other drawings from these accompanying drawings without creative efforts.
FIG. 1 is a schematic structural diagram of a shunt for treating hydrocephalus according to Embodiment 1 of the present disclosure;
FIG. 2 is a left view of FIG. 1;
FIG. 3 is a right view of FIG. 1;
FIG. 4 is a schematic structural diagram of a system for treating hydrocephalus according to Embodiment 1 of the present disclosure;
FIG. 5 is a schematic structural diagram of a puncture member according to Embodiment 1 of the present disclosure;
FIG. 6 is a schematic structural diagram of a puncture member at a distal end of a shunt according to Embodiment 1 of the present disclosure;
FIG. 7 is a schematic structural diagram of a shunt for treating hydrocephalus according to Embodiment 2 of the present disclosure;
FIG. 8 is a schematic structural diagram of a system for treating hydrocephalus according to Embodiment 2 of the present disclosure;
FIG. 9 is a schematic structural diagram of a system for treating hydrocephalus in use according to Embodiment 2 of the present disclosure;
FIG. 10 is a schematic structural diagram of a system for treating hydrocephalus according to Embodiment 3 of the present disclosure;
FIG. 11 is a schematic structural diagram of a puncture member having a tubular main body and a flat tip in a system for treating hydrocephalus according to Embodiment 3 of the present disclosure;
FIG. 12 is a schematic structural diagram of a puncture member having both a flat main body and a flat tip in a system for treating hydrocephalus according to Embodiment 3 of the present disclosure;
FIG. 13 is a schematic structural diagram of a tip of a puncture member in a system for treating hydrocephalus according to Embodiment 3 of the present disclosure;
FIG. 14 is a schematic structural diagram of a system for treating hydrocephalus according to Embodiment 4 of the present disclosure;
FIG. 15 is a schematic structural diagram of a system for treating hydrocephalus according to Embodiment 5 of the present disclosure; and
FIG. 16 is a size comparison diagram of a puncture needle and a puncture opening according to Embodiment 5 of the present disclosure.

Reference numerals: 1: shunt, 11: tube body, 12: proximal portion, 13: distal portion, 14: limiting portion, 15: flow lumen, 16: rounded rectangular cross-sectional structure, 17: circular tube cross-sectional structure, 2: delivery catheter, 3: shunt pusher, 4: puncture member, 5: guide member, 6: main body, 7: tip, and 8: puncture protective sheath.

### DETAILED DESCRIPTION OF THE EMBODIMENTS

The technical solutions in the implementations of the present disclosure are clearly and completely described below with reference to the drawings of the implementations of the present disclosure. Apparently, the described implementations are merely some rather than all of the implementations of the present disclosure. On the basis of the implementations of the present disclosure, all other implementations obtained by a person of ordinary skill in the art without making creative efforts shall fall within the protection scope of the present disclosure.

In all the following embodiments, the radial size refers to a length, a width, a diameter, an area, and the like in a radial direction of the shunt. Since a dura mater and an arachnoid mater are usually tightly connected together in a human body, in the present disclosure, the term "dura mater" is used to refer to both the dura mater and the arachnoid mater in order to avoid overly lengthy description.

### Embodiment 1:

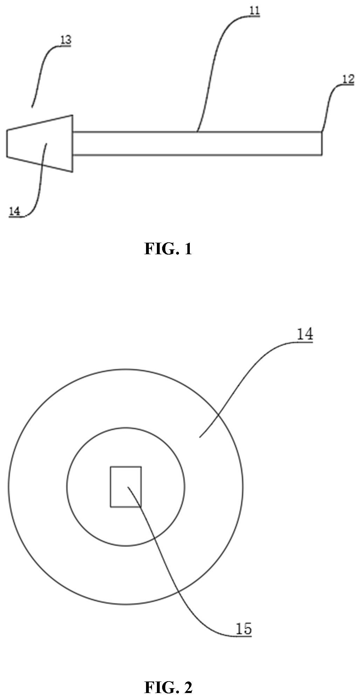

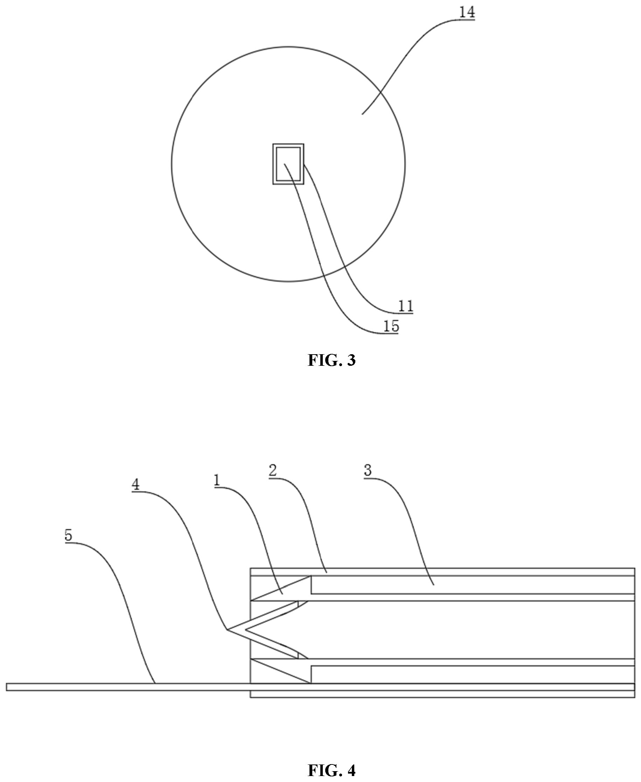

As shown in FIG. 1 to FIG. 5, a shunt and system for treating hydrocephalus are provided. The system includes a shunt 1 and a delivery system configured to deliver the shunt 1 to a designated position in a blood vessel. During delivery, the shunt 1 is placed inside the delivery system. After reaching the designated position, the shunt 1 is disposed at the designated position through the delivery system. The dura mater between the ventricular system and the venous system is punctured by a puncture member of the delivery system to form a puncture opening.

The shunt 1 includes a distal portion 13 provided with a cerebrospinal fluid inlet, a proximal portion 12 provided with a cerebrospinal fluid outlet, and a tube body 11 provided with a flow lumen 15. The distal portion 13 is configured to be deployed in a patient's cistern, the proximal portion 12 is configured to be deployed in or near the patient's jugular vein, the cerebrospinal fluid inlet communicates with the cerebrospinal fluid outlet through the flow lumen 15, and the tube body 11 is connected to the distal portion 13 and the proximal portion 12. When the distal portion 13 is deployed in the cistern and the proximal portion 12 is deployed in or near the jugular vein, cerebrospinal fluid in the cistern is capable of being drained into the jugular vein through the cerebrospinal fluid inlet, the flow lumen 15, and the cerebrospinal fluid outlet. One or more cerebrospinal fluid inlets and cerebrospinal fluid outlets may be provided, and the quantity may be determined according to actual needs.

The distal portion 13 has a limiting portion 14 configured to be in contact with the dura mater. A maximum radial size of the limiting portion 14 in both the delivery state and the deployed state is greater than a minimum radial size of the puncture opening and a minimum radial size of the tube body 11. In this embodiment, the limiting portion 14 is configured as a truncated cone structure. A maximum limiting outer diameter of the truncated cone structure is 0.9 mm. A cross-sectional area of a distal end of the truncated cone structure is smaller than that of a proximal end of the truncated cone structure. The cross-sectional area of the truncated cone structure gradually increases from the distal end to the proximal end. The cross-sectional area of the distal end of the truncated cone structure is set close to a size of the puncture opening.

The distal portion 13 is provided with an expanding portion connected to the limiting portion 14. The expanding portion is farther from the proximal portion 12 than the limiting portion. A radial size of the expanding portion gradually increases in a direction from the distal portion 13 toward the proximal portion 12. A minimum radial size of the expanding portion is less than or equal to a maximum radial size of the puncture opening. In this embodiment, the truncated cone structure whose cross-sectional area gradually increases from the distal end to the proximal end forms the expanding portion. The cross-sectional area of the distal end of the truncated cone structure is set close to the size of the puncture opening, facilitating an entry of the truncated cone structure into the puncture opening to dilate the puncture opening. The truncated cone structure serves to dilate the puncture opening, to limit and fasten the distal portion 13 of the shunt on the cistern side, and to provide protection, that is, to prevent the puncture member from cutting and damaging tissue or instruments.

A maximum radial size of the distal end of the tube body 11 is greater than a minimum radial size of the puncture opening, so that the distal end of the tube body 11 is capable of achieving better sealing with the puncture opening. In this embodiment, the tube body 11 is configured as a rounded rectangular cross-sectional structure. The flow lumen 15 is a through-lumen having a cross-sectional size of 0.25 mm × 0.14 mm. The size of the flow lumen 15 is determined by a required flow rate through the through-lumen, and here the flow rate of the flow lumen 15 is less than 20 ml/h.

In clinical operation, the puncture opening formed by the puncture member is mostly an irregularly shaped hole with a non-uniform radial size. A difference between the maximum radial size of the limiting portion 14 and the minimum radial size of the puncture opening is a maximum limiting size. A difference between a minimum radial size of the limiting portion 14 and the maximum radial size of the puncture opening is a minimum limiting size. The maximum limiting size and the minimum limiting size range from 0 mm to 3 mm. In this way, the limiting portion 14 is capable of being well limited within the puncture opening, including an irregularly shaped hole, achieving fastening and limitation of the distal portion 13.

In this embodiment, a central axis of the limiting portion 14 coincides with a central axis of the tube body 11. In other embodiments, the limiting portion 14 may be disposed to make its central axis do not coincide with the central axis of the tube body 11. The noncoincident disposition allows the limiting portion to pass through the puncture opening with a smaller size while forming a limiting fit with a larger difference in sizes between the limiting portion and the puncture opening.

In this embodiment, a radiopaque marker is provided on a surface of the truncated cone structure, or the truncated cone structure is marked with a radiopaque marker material, or a radiopaque marker ring is provided at the distal end of the truncated cone structure.

In other embodiments, the distal portion 13 may also be provided with other external structures that are configured to provide the limiting portion and the expanding portion, including but not limited to a truncated cone, an ellipsoid, a prism, a cylinder, a cone, and the like. The truncated cone, ellipsoid, prism, cylinder, and cone have a smaller distal end, facilitating passage through the puncture opening.

The distal end shape of the tube body 11 may be circular or flat, including but not limited to circular, rectangular, prismatic, elliptical, crescent-shaped, trapezoidal, meniscus, and other extendable shapes such as wave-shaped, double-wave-shaped, bullet-headed, right-angled trapezoidal, and the like. Compared with a circular distal end of the tube body, a flat distal end of the tube body is capable of providing a larger limiting size as much as possible, further optimizing the limitation The tube body may also be provided with a distal end shape that fits the puncture opening.

Specifically, the shunt 1 is provided with a flow direction control member that is configured to allow cerebrospinal fluid to flow only from the cistern into the venous system (particularly in or near the jugular vein) through the inner lumen of the shunt. The flow direction control member may be configured as a one-way valve, a unidirectional flow channel, a unidirectional flow surface, or a spring valve. The one-way valve may be configured as a duckbill valve, a slit valve, a bicuspid valve, a monocuspid valve, and the like. The unidirectional flow channel may be configured as a Tesla valve. The unidirectional flow surface may be configured as a unidirectional flow microstructure, a hydrophilic-hydrophobic alternating structure, or a unidirectional damping-increasing surface structure.

Preferably, the flow direction control member is disposed at the proximal end of the shunt and within the flow lumen 15 of the shunt 1. In other embodiments, the flow direction control member may be disposed at the distal end of the shunt or within the flow lumen 15 as needed.

The proximal end of the shunt may also be provided with a shielding protection device configured to isolate endothelial cells of the venous system from the proximal outlet of the shunt, including but not limited to a stent, a balloon, a device similar to the distal end, a membrane, and the like.

The limiting portion 14 includes an elastic structure. The elastic structure is a structure that is capable of contracting under force, including but not limited to a spring, a stent, a wire, a coil, and the like. The elastic structure is located at the distal end of the limiting portion 14. In this way, when passing through the puncture opening, the distal end is compressed by the dura mater, and the dura mater also undergoes elastic deformation. This minimizes enlargement of the puncture opening. Alternatively, the limiting portion 14 may be made of an elastic material, including but not limited to a spring, a stent, a wire, a coil, and the like.

A sealant is provided between the shunt 1 and the dural puncture opening, configured to repair the puncture opening, including but not limited to degradable hydrogel, medical glue, and the like, and including but not limited to natural-component sealants (for example, fibrinbased glues), semi-synthetic glues (for example, gelatin-based and albumin-based glues), fully synthetic glues (for example, acrylate-based and polyethylene glycol-based glues), and combinations thereof.

Polyethylene glycol-based glues include but are not limited to four-arm polyethylene glycol (for example, four-arm polyethylene glycol succinimidyl glutarate) and derivatives thereof, eight-arm polyethylene glycol and derivatives thereof, and combinations thereof.

The shunt 1 is further provided with a semipermeable membrane, and is configured to restrict flow, diffusion, exchange, and the like, of components between the cistern and the venous system, including but not limited to a PTFE porous membrane, an ePTFE porous membrane, and the like. The semipermeable membrane is disposed in the distal portion 13, the proximal portion 12, or the tube body 11. Specifically, the semipermeable membrane is disposed in the flow lumen 15 of the shunt 1, configured to make CSF flow through the cerebrospinal fluid inlet of the shunt, the semipermeable membrane, and the cerebrospinal fluid outlet. Alternatively, the semipermeable membrane is disposed at the cerebrospinal fluid inlet, the cerebrospinal fluid outlet, or both.

The shunt further includes a polymer liner disposed on the tube body 11, the proximal portion 12, and the distal portion 13. Specifically, the polymer liner is disposed on a part or all of the external surface and the internal luminal surface of the shunt 1. The polymer liner is selected from a material that minimizes protein and/or cell adhesion. More specifically, the polymer liner material is PTFE, PET, and the like. Further, the polymer liner is disposed on the external surface of the shunt 1 and/or the inner surface of the flow lumen 15.

The shunt further includes an antithrombogenic layer disposed on the tube body 11, the proximal portion 12, and the distal portion 13. Specifically, the antithrombogenic layer is disposed on a part or all of the external surface and the internal luminal surface of the shunt 1. The antithrombogenic layer may be an antithrombogenic coating, a layer having an antithrombogenic component or antithrombogenic structure, or a combination thereof.

The delivery system includes a delivery catheter 2, a shunt pusher 3, a puncture member 4, and a guide member 5. During delivery, the shunt 1, the shunt pusher 3, the puncture member 4, and the guide member 5 are located within a lumen of the delivery catheter 2. The puncture member 4 is disposed outside the shunt 1, inside the shunt 1 or on the distal portion 13. The delivery catheter 2 is configured to deliver the puncture member 4 and the shunt 1 to the designated position. The guide member 5 is configured to guide movement of the delivery catheter 2 in the venous system. The shunt pusher 3 is configured to push the shunt 1 along the delivery catheter 2. The puncture member 4 is configured to move along the delivery catheter 2 to the designated position and then puncture the dura mater to form the puncture opening.

The guide member 5 may be configured to include, but is not limited to, a guidewire, a distally steerable microcatheter, a guiding catheter, a catheter with a unilateral balloon, a "black mudfish" guidewire, a contrast catheter, a stent or balloon with an inner lumen. In this embodiment, the shunt pusher 3 is configured as a shunt push rod, and the guide member 5 is configured as a guide rod. The shunt 1, the shunt push rod, the puncture member 4, and the guide rod are all disposed inside the delivery catheter 2. The delivery catheter 2 is provided with an inner lumen. The inner lumen may include a lumen accommodating the shunt 1 and a lumen accommodating the guide rod.

In this embodiment, the puncture member 4 is disposed in the flow lumen 15. The puncture member 4 includes a main body and a puncture tip having a cutting edge at a distal end. The distal end of the main body is connected to the puncture tip. The main body of the puncture member includes a distal end and a proximal end. The distal end of the main body of the puncture member is configured as a hypotube structure, a braided structure, and the like. The puncture member is configured as a circular or flat thin blade. Specifically, the puncture tip is configured to puncture a dura mater to form the puncture opening. The cross-sectional shape of the puncture tip may be circular (or a non-closed slotted circle) or a flat thin blade, including but not limited to circular, rectangular, prismatic, elliptical, crescent-shaped, trapezoidal, meniscus, and other extendable shapes including wave-shaped, double-wave-shaped, bullet-headed, right-angled trapezoidal, and the like. When the puncture tip is configured as circular, since most conventional puncture needles are circular tips, they are relatively convenient to use and are directly applied in the present disclosure. Preferably, the puncture tip is configured as a flat thin blade. The puncture opening formed by puncturing the dura mater has a small radial size, thereby providing a larger limiting size as much as possible.

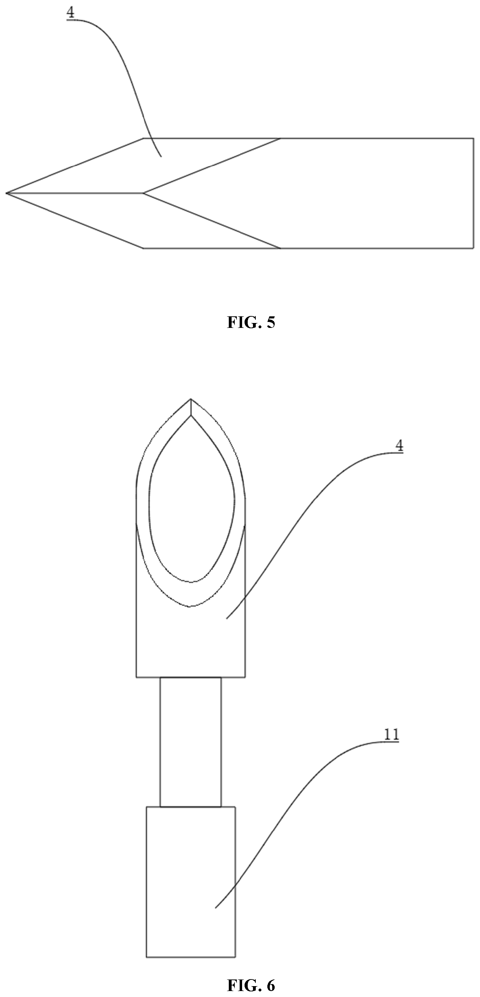

In this embodiment, the puncture tip is configured as a flat thin blade, as shown in FIG. 5. The flat thin blade has a rectangular shape corresponding to the flow lumen, with a cross-sectional size of 0.3 mm × 0.1 mm. The distal end of the flat thin blade is provided with two cutting edges.

In other embodiments, relative positions of the puncture member 4 and the shunt 1 include, but are not limited to, the puncture member being located outside the shunt tube, inside the shunt tube, or at the distal end of the shunt.

In this embodiment, the shunt pusher is configured as a tubular structure. The middle of the tubular structure has a rectangular cavity corresponding to the flow lumen 15 of the shunt 1. The shunt 1 is disposed in the rectangular cavity of the shunt pusher, and the distal portion of the shunt 1 is located at one end of the shunt pusher.

The delivery system includes an expandable anchoring member connected to the guide member. The expandable anchoring member includes, but is not limited to, a balloon, a stent, and the like. During delivery, the guide member is connected to the expandable anchoring member located distal to the inferior petrosal sinus.

The delivery system further includes a dilation member configured to move along the guide member and/or the puncture member and/or the shunt 1 to dilate the puncture opening formed by the puncture member.

The delivery system includes at least one radiopaque marker. A position and a size of the radiopaque marker are set to indicate a trajectory of the puncture tip. Specifically, the distal end of the puncture member is provided with a radiopaque marker.

The delivery system further includes an operating handle. A part or all of the proximal ends of the components of the delivery system are connected to the operating handle, and the delivery system is operated by the operating handle.

The process of using the delivery system is as follows:
The femoral vein is punctured to establish an interventional access route. The guide member of the delivery system, including a guiding catheter, is advanced to the internal jugular vein. The following four manners may then be used to advance the delivery system into the inferior petrosal sinus.

Manner (1): Under dual C-arm anteroposterior and lateral venous phase roadmap, a microcatheter is guided by a micro-guidewire into the inferior petrosal sinus.

Manner (2): A single-curve angiographic catheter is guided by a "black mudfish" guidewire to dock with the inferior petrosal sinus. The "black mudfish" guidewire is withdrawn. Under anteroposterior roadmap of the common carotid artery, the microcatheter is guided by a micro-guidewire through the angiographic catheter to super-select the inferior petrosal sinus, advancing about 30 mm from an exit of the internal jugular vein catheter. The micro-guidewire is withdrawn and the expandable anchoring member of the delivery system is advanced. The anchoring member is carried by the microcatheter to a depth of about 30 mm.

Manner (3): In the case of a narrow entrance of the inferior petrosal sinus: An exchange-type mudfish guidewire is advanced into the guiding catheter. The guiding catheter is withdrawn and exchanged for a single-curve angiographic catheter. With the strong support of the angiographic catheter, the mudfish guidewire is selected into the inferior petrosal sinus. The angiographic catheter is withdrawn and the guiding catheter and a multi-function angiographic catheter are coaxially exchanged. The multi-function angiographic catheter is configured to pass the narrowing of the inferior petrosal sinus and bring the guiding catheter into the inferior petrosal sinus. A microcatheter is advanced along the guiding catheter. The expandable anchoring member-anchoring stent is carried by the microcatheter to a depth of about 30 mm. The microcatheter is withdrawn.

Manner (4): In the case where the inferior petrosal sinus is not visible, the entrance of the inferior petrosal sinus is explored using a contrast guidewire or a mudfish guidewire, then the microcatheter is guided into the inferior petrosal sinus according to the above operations to deploy the stent.

The process of delivering the shunt 1 to the designated position in this embodiment is as follows: The delivery catheter is advanced along the guide member into the inferior petrosal sinus. Under the pushing action of the shunt pusher, the shunt 1 is advanced along the inner lumen of the delivery catheter into the inferior petrosal sinus. Imaging is used to determine whether the position of the distal truncated cone structure of the shunt is appropriate. If the position of the distal truncated cone structure is appropriate, the proximal end of the puncture member is pushed forward to puncture the dura mater and arachnoid mater to form the puncture opening and enter the cistern. The shunt pusher is pushed, and the distal truncated cone structure of the shunt is moved forward along the puncture member. The dural puncture opening is compressed by the truncated cone structure, and the dural puncture opening is gradually dilated by the expanding portion of the truncated cone structure to enter the cistern. The shunt pusher is withdrawn, and the tissue around the dural puncture opening is resiliently recoiled to compress the tube body of the shunt 1. The delivery catheter and the guide member are then withdrawn sequentially. Because the tube body is larger in size than the puncture opening, the tube body forms a seal with the puncture opening. Because the limiting portion has an occupied size relative to the puncture opening, the limiting portion forms a snap-fit with the puncture opening.

When the shunt 1 is deployed in the venous system, with the distal portion of the shunt 1 disposed in the cistern and the proximal portion of the shunt 1 disposed in or near the jugular vein, cerebrospinal fluid flows from the cerebellopontine angle cistern into the jugular vein through the one or more cerebrospinal fluid inlets at the distal end of the shunt, the flow lumen of the shunt, and the cerebrospinal fluid outlet at the proximal end of the shunt.

### Embodiment 2:

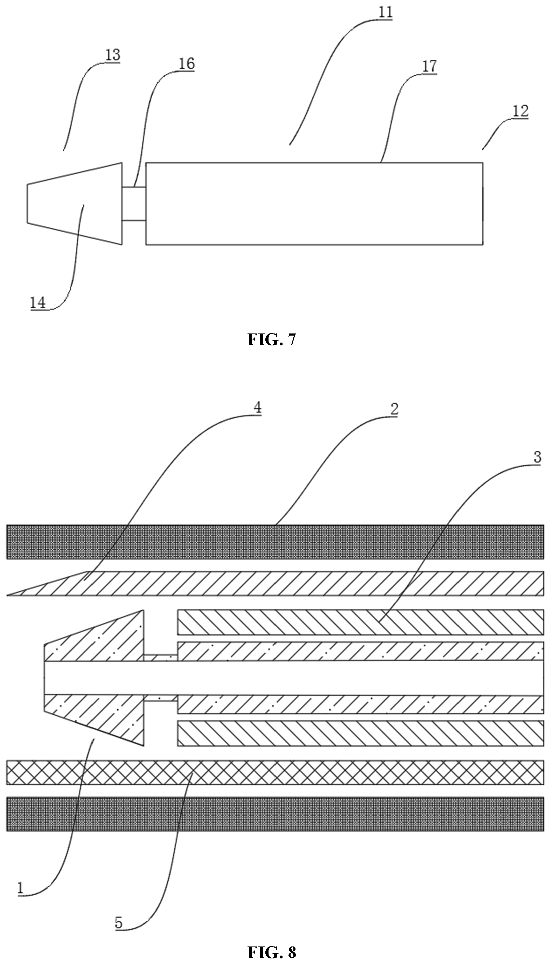

As shown in FIG. 1 to FIG. 3, FIG. 7, and FIG. 8, a shunt and system for treating hydrocephalus are provided. The system includes a shunt 1 and a delivery system configured to deliver the shunt 1 to a designated position in a blood vessel.

Other structures are the same as those in Embodiment 1. The shunt 1 in this embodiment differs from that in Embodiment 1 in that the tube body 11 includes a distal rounded rectangular cross-sectional structure 16 and the remaining circular tube cross-sectional structure 17. The rounded rectangular cross-sectional structure has a size of 0.6 mm* 0.4 mm. The tube body has a flow lumen 15 thereinside. The flow lumen 15 is a through-lumen having a size of 0.25 mm*0.14 mm. The size of the through-lumen is determined by a required flow rate, which is less than 20 ml/h. The tube body 11 is provided with a stepped surface close to the distal portion 13. That is, a stepped surface is formed between the distal end of the tube body and the remaining portion. The stepped surface is configured to form limitation with a side of the dura mater away from the ventricular system, preventing the shunt from moving excessively into the cistern, and preventing the shunt 1 from undergoing large displacement after deployment. A distal tube segment having a minimum radial size smaller than a maximum radial size of the tube body 11 is provided between the stepped surface and the limiting portion 14. In this embodiment, the stepped surface is formed between the rounded rectangular cross-sectional structure 16 of the tube body 11 and the remaining circular tube cross-sectional structure 17. The rounded rectangular cross-sectional structure 16 serves as the distal tube segment, and a partial radial size of the rounded rectangular cross-sectional structure 16 is smaller than the radial size of the remaining circular tube cross-sectional structure 17.

The delivery system in this embodiment differs from that in embodiment 1 in that the puncture member is disposed outside the shunt 1 and disposed in parallel with the shunt 1. The puncture member is configured as a tubular or flat structure. A cross-sectional size of the puncture member is set to 0.3 mm*0.1 mm. The puncture tip is a flat thin blade provided with two cutting edges. Specifically, the puncture member is a puncture needle. The shunt pusher is configured as a shunt push rod. The shunt push rod is located on a lateral side or at the proximal end of the shunt 1, and is capable of pushing the distal end or the proximal end of the shunt to advance the shunt. The anchoring member is configured as an anchoring stent.

The delivery system includes a delivery catheter, a shunt push rod, a puncture needle, a guide rod, and an anchoring stent. The shunt 1, the delivery catheter, the shunt push rod, the puncture needle, and the guide rod are all disposed inside the delivery catheter. The anchoring stent is connected to distal end of the guide rod, and the anchoring stent is configured to be deployed distal to the inferior petrosal sinus.

The guide member may also be configured to include, but is not limited to, a guidewire, a distally steerable microcatheter, a guiding catheter, a catheter with a unilateral balloon, a stent or balloon with an inner lumen, and the like.

A process for delivering the shunt 1 to the actuation position using the delivery system in this embodiment is as follows:

The delivery catheter is advanced along the guide rod into the inferior petrosal sinus. Under the action of the proximal end of the puncture member and the shunt push rod, the shunt 1 and the puncture member are advanced along the inner lumen of the delivery catheter into the inferior petrosal sinus. Imaging is used to determine whether the position of the distal truncated cone structure of the shunt 1 is appropriate. If the position of the truncated cone structure is appropriate, the proximal end of the puncture member is pushed forward to puncture the dura mater and arachnoid mater to form the puncture opening and enter the cistern. The shunt push rod is pushed, and the distal truncated cone structure of the shunt moves forward along the puncture member. The dural puncture opening is compressed by the truncated cone structure, and the dural puncture opening is gradually dilated by the truncated cone structure to enter the cistern. The shunt push rod is withdrawn, and the tissue around the dural puncture opening is resiliently recoiled to compress the tube body of the shunt 1. The delivery catheter and the guide rod are then withdrawn sequentially. Because the distal end of the tube body that fits the puncture opening is larger in size than the puncture opening, the tube body forms a seal with the puncture opening. Because the truncated cone structure has an occupied size relative to the puncture opening, the limiting portion forms a snap-fit with the puncture opening. Because the stepped surface between the distal end of the tube body and the circular tube has a size larger than the puncture opening, the stepped surface forms a limit with the puncture opening.

### Embodiment 3:

A shunt and system for treating hydrocephalus are provided. The system includes a shunt 1 and a delivery system configured to deliver the shunt 1 to a designated position in a blood vessel.

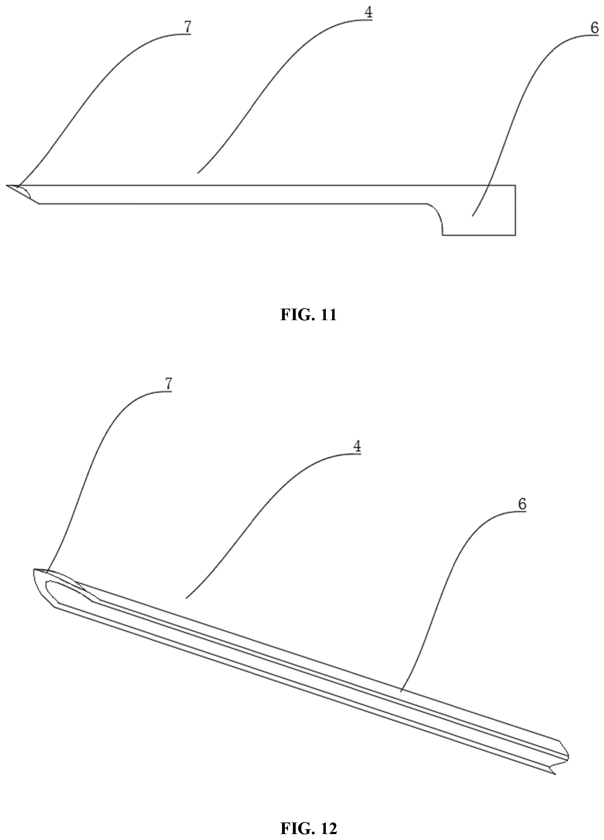

Other structures are the same as those in embodiment 2. The delivery system in this embodiment differs from that in embodiment 2 in that the puncture member 4 includes a main body 6 and a tip 7. The main body 6 of the puncture member 4 is configured as a tubular, flat, or non-closed slotted tubular shape, and the like. A cross-sectional shape of the tip 7 of the puncture member 4 is a meniscus flat shape. The tip 7 is provided with a cutting edge, and the cutting edge is disposed relatively closer to the axis of the shunt 1, as shown in FIG. 11, FIG. 12, and FIG. 13. In this way, the puncture member is capable of occupying less space within the delivery catheter, and the puncture opening formed has a smaller radial size, thereby providing a larger limiting size.

Specifically, the meniscus flat puncture member tip is a puncture needle having an outer diameter of 0.9 mm and an angular wall thickness of 0.1 mm. The puncture opening formed by such a puncture needle is less than 0.64 mm in diameter. The puncture needle is pre-shaped to be able to move forward biased toward the axis of the shunt 1, or the truncated cone structure of the shunt 1 is tilted toward the puncture needle. The proximal portion of the main body of the puncture needle is a hypotube structure.

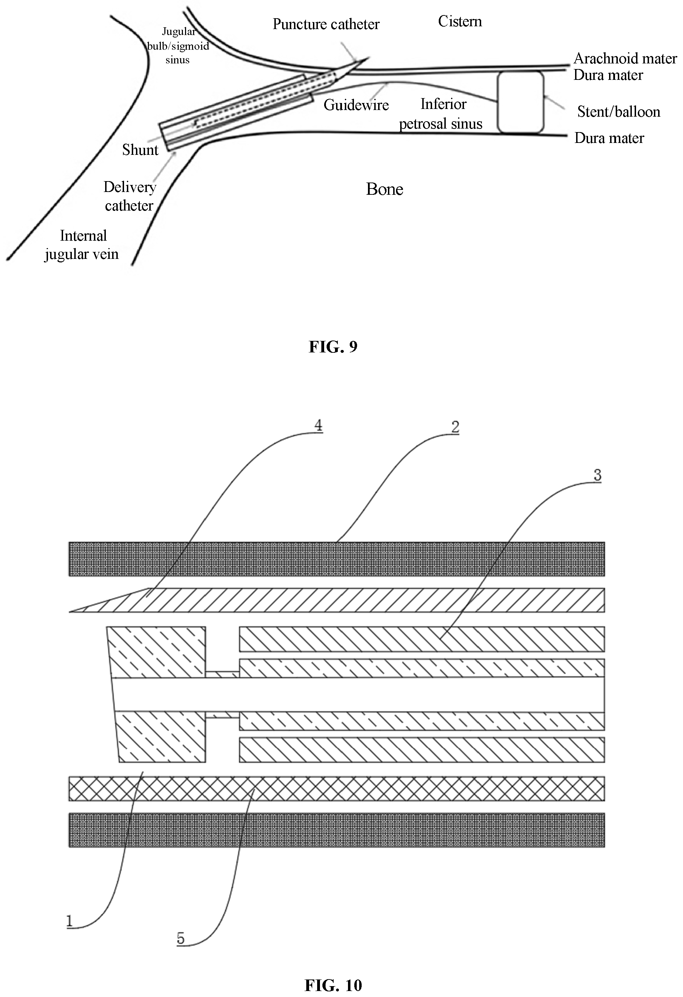

As shown in FIG. 10, the delivery system includes a delivery catheter 2, a shunt pusher, a puncture member 4, and a guide rod 5. The shunt 1, the shunt pusher, the puncture member 4, and the guide rod 5 are all disposed inside the delivery catheter 2. The delivery catheter 2 is provided with lumens, including a lumen accommodating the puncture member 4, a lumen portion accommodating the shunt 1, and a lumen portion accommodating the guide rod 5. The puncture member 4 is located on one side between the shunt 1 and the delivery catheter 2. The shunt pusher is outside the shunt 1. The guide rod 5 is located in a corresponding delivery lumen.

A process for delivering the shunt 1 to the actuation position using the delivery system in this embodiment is as follows:
The delivery catheter 2 is advanced along the guide rod 5 into the inferior petrosal sinus. Under the action of the proximal end of the puncture member and the shunt pusher, the shunt 1 and the puncture member 4 are advanced along the inner lumen of the delivery catheter 2 into the inferior petrosal sinus. Imaging is used to determine whether the position of the distal truncated cone structure of the shunt 1 is appropriate. If the position of the truncated cone structure is appropriate, the proximal end of the puncture needle is pushed forward to puncture the dura mater and arachnoid mater to form the puncture opening and enter the cistern. The shunt pusher is pushed, and the distal truncated cone structure of the shunt moves forward along the puncture member. The dural puncture opening is compressed by the truncated cone structure, and the dural puncture opening is gradually dilated by the expanding portion of the truncated cone structure to enter the cistern. The shunt pusher is withdrawn, and the tissue around the dural puncture opening is resiliently recoiled to compress the tube body of the shunt 1. The delivery catheter 2 and the guide rod 5 are then withdrawn sequentially. Because the distal end of the tube body that fits the puncture opening is larger in size than the puncture opening, the tube body forms a seal with the puncture opening. Because the truncated cone structure has an occupied size relative to the puncture opening, the limiting portion forms a snap-fit with the puncture opening. Because the stepped surface between the distal end of the tube body and the circular tube has a size larger than the puncture opening, the stepped surface forms a limit with the puncture opening.

### Embodiment 4:

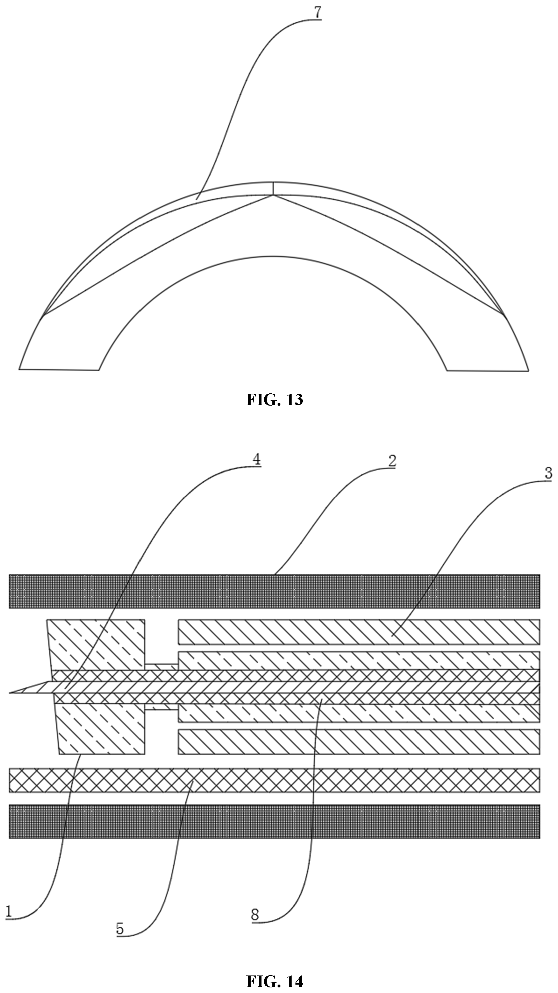

As shown in FIG. 14, a shunt and system for treating hydrocephalus are provided. The system includes a shunt 1 and a delivery system configured to deliver the shunt 1 to a designated position in a blood vessel.

Other structures are the same as those in Embodiment 3. The delivery system in this embodiment differs from that in Embodiment 3 in that the puncture member is located inside the shunt 1, and a puncture protective sheath 8 is provided between the entire inner wall of the lumen of the shunt 1 that is configured to accommodate the puncture member and the puncture member.

### Embodiment 5:

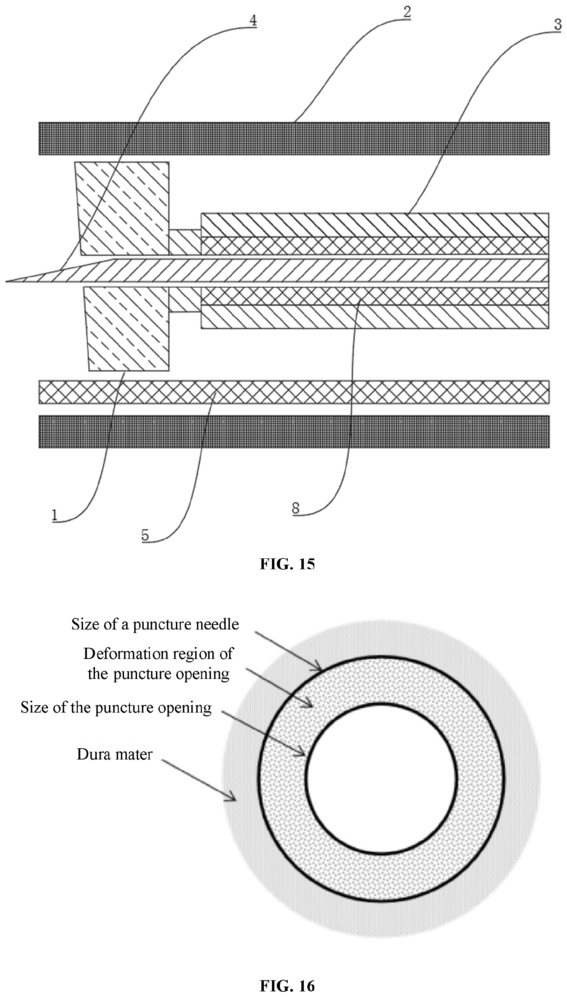

As shown in FIG. 15, a shunt and system for treating hydrocephalus are provided. The system includes a shunt 1 and a delivery system configured to deliver the shunt 1 to a designated position in a blood vessel.

Other structures are the same as those in Embodiment 4. The delivery system in this embodiment differs from that in Embodiment 4 in that the puncture member 4 is located inside the shunt 1, a puncture protective sheath 8 is provided between a portion of the inner wall of the lumen of the shunt 1 that is configured to accommodate the puncture member and the puncture member 4, and the puncture protective sheath 8 also serves as a shunt pusher.

Experimental tests in the present disclosure have shown that the dural puncture opening is mainly meniscus-shaped, crescent-shaped, or arc-shaped. Therefore, the puncture opening undergoes substantial compressive elastic deformation when compressed by the tube body. Moreover, the size of the puncture opening formed in the dura mater is smaller than the diameter of the puncture member used. The dura mater has specific elastic deformation space. Therefore, after the shunt 1 passes through the puncture opening, limitation and snap-fit can be achieved due to the elastic deformation of the dura mater. In addition, even if the puncture opening is torn beyond elastic deformation, because the dura mater is a soft membrane material, the puncture opening is capable of being dilated by tube body of a larger size, thereby further closing a torn position.

For example, using a puncture needle with an outer diameter of 0.9 mm and a willow-leaf blade, the obtained puncture opening has a size of 0.45 mm. Using a puncture needle with an outer diameter of 0.9 mm and a half-pointed tip, the obtained puncture opening has a size less than 0.6 mm. Using a truncated cone at the distal end of the shunt that is larger than the outer diameter of the puncture needle, and a tube body that is slightly larger than the puncture opening, fixation and sealing of the shunt 1 can be achieved. In addition, calculations show that a force on the shunt 1 in the venous system is very small, thus requiring a low anchoring force and consequently imposing low requirements on a size of the distal limiting portion. A comparison between a size of the puncture needle tip and a size of the puncture opening is shown in FIG. 16. Taking a circular puncture tip and puncture opening as an example, because the dura mater has a specific elastic deformation capability, when the dura mater is punctured by the puncture tip of the puncture member, a puncture opening having a radial size smaller than the radial size of the puncture tip is formed in the dura mater.

When the puncture tip of the puncture member in the present disclosure is configured as a thin blade, the puncture member of this structure can fully utilize the space of the flow lumen of the shunt 1 while maximizing the occupied size. At the same time, because the puncture blade has the smallest size and the tube body is large, sealing with the tube body can be achieved. In addition when the puncture needle protective sheath is disposed within the flow lumen 15 of the shunt 1, the puncture needle protective sheath can serve as a shunt pusher while providing protection for the inner liner of the shunt 1, preventing the puncture needle from cutting and damaging the inner wall of the flow lumen 15 of the shunt 1 and the flow direction control member.

Finally, it should be noted that the above descriptions are only preferred embodiments of the present disclosure, and are not intended to limit the present disclosure. The examples of the embodiments, where parts not described in detail, are common general knowledge of those of ordinary skill in the art. For those skilled in the art, they may still modify the technical solutions described in the foregoing embodiments, or make equivalent substitutions to some of the technical features thereof. Any modification, equivalent substitution, improvement, and the like within the spirit and principles of the present disclosure shall fall within the scope of protection of the present disclosure.

## Claims

1. A shunt for treating hydrocephalus, configured to be deployed in a ventricular system and a venous system of a patient to drain cerebrospinal fluid from the ventricular system of the patient into the venous system of the patient, comprising: a distal portion (13) provided with a cerebrospinal fluid inlet, a proximal portion (12) provided with a cerebrospinal fluid outlet, and a tube body (11) provided with a flow lumen, wherein a dura mater between the ventricular system and the venous system is provided with a puncture opening, the distal portion (13) is configured to be disposed in the ventricular system, the proximal portion (12) is configured to be disposed in the venous system, the distal portion (13) is connected to the proximal portion (12) through the tube body (11), the cerebrospinal fluid inlet communicates with the cerebrospinal fluid outlet through the flow lumen, the distal portion (13) is provided with a limiting portion (14) configured to be in contact with the dura mater, and a maximum radial size of the limiting portion (14) in a delivery state is greater than both a minimum radial size of the puncture opening and a minimum radial size of the tube body (11).

2. The shunt for treating hydrocephalus according to claim 1, wherein a maximum radial size of a distal end of the tube body (11) is greater than the minimum radial size of the puncture opening.

3. The shunt for treating hydrocephalus according to claim 1, wherein the distal portion (13) is provided with an expanding portion connected to the limiting portion (14), the expanding portion is farther from the proximal portion (12) than the limiting portion (14), and a radial size of the expanding portion gradually increases in a direction from the distal portion (13) to the proximal portion (12).

4. The shunt for treating hydrocephalus according to claim 3, wherein a minimum radial size of the expanding portion is less than or equal to a maximum radial size of the puncture opening.

5. The shunt for treating hydrocephalus according to claim 1, wherein the limiting portion (14) is in a shape of a truncated cone, an ellipsoid, a prism, a cylinder, or a cone; and/or
a distal end of the tube body (11) close to the distal portion (13) is circular or flat; and/or
a central axis of the limiting portion (14) coincides with or does not coincide with a central axis of the tube body (11).

6. The shunt for treating hydrocephalus according to claim 1, wherein the limiting portion (14) is made of an elastic material; or
the limiting portion (14) comprises an elastic structure, and the elastic structure is located at a distal end of the limiting portion (14).

7. The shunt for treating hydrocephalus according to claim 1, wherein a difference between the maximum radial size of the limiting portion (14) and the minimum radial size of the puncture opening is a maximum limiting size, a difference between a minimum radial size of the limiting portion (14) and a maximum radial size of the puncture opening is a minimum limiting size, and the maximum limiting size and the minimum limiting size range from 0 mm to 3 mm.

8. The shunt for treating hydrocephalus according to claim 1, wherein the tube body (11) is provided with a stepped surface close to the distal portion (13), the stepped surface is configured to form limitation with a side of the dura mater away from the ventricular system, and a distal tube segment having a minimum radial size smaller than a maximum radial size of the tube body (11) is provided between the stepped surface and the limiting portion (14).

9. The shunt for treating hydrocephalus according to claim 1, wherein a sealant is provided between the tube body (11) and the puncture opening.

10. The shunt for treating hydrocephalus according to claim 1, further comprising a flow direction control member, wherein the flow direction control member is configured to allow the cerebrospinal fluid to flow only from the ventricular system into the venous system through the flow lumen, and the flow direction control member is disposed in the flow lumen, or on the proximal portion (12), or on the distal portion (13), or a combination thereof.

11. The shunt for treating hydrocephalus according to claim 10, wherein the flow direction control member is a one-way valve, a unidirectional flow channel, a unidirectional flow surface, or a spring valve.

12. The shunt for treating hydrocephalus according to claim 11, wherein the one-way valve is a duckbill valve, a slit valve, a bicuspid valve, or a monocuspid valve; the unidirectional flow channel is a Tesla valve; and the unidirectional flow surface is disposed as a unidirectional flow microstructure, a hydrophilic-hydrophobic alternating structure, or a unidirectional damping-increasing surface structure.

13. The shunt for treating hydrocephalus according to claim 1, wherein a semipermeable membrane is provided in the distal portion (13), the proximal portion (12), or the tube body (11), and the semipermeable membrane is configured to restrict flow, diffusion, or exchange of some components between the ventricular system and the venous system; and/or
a polymer liner and/or an antithrombogenic coating is further comprised, wherein the polymer liner and/or the antithrombogenic coating is disposed on the tube body (11), the proximal portion (12), and the distal portion (13); and/or
the proximal portion (12) is provided with a shielding protection device, and the shielding protection device is a stent, a balloon, or a membrane; and/or
at least one radiopaque marker is further comprised.

14. A system for treating hydrocephalus, comprising: the shunt according to any one of claims 1 to 13 and a delivery system, wherein the delivery system is configured to deliver the shunt to designated positions in the venous system and the ventricular system, and during delivery, the shunt is placed inside the delivery system.

15. The system for treating hydrocephalus according to claim 14, wherein the delivery system comprises a delivery catheter (2), a shunt pusher (3), a puncture member (4), and a guide member (5), wherein during delivery, the shunt, the shunt pusher (3), the puncture member (4), and the guide member (5) are all located inside the delivery catheter (2), and the puncture member (4) is disposed outside the shunt, inside the shunt, or on the distal portion.

16. The system for treating hydrocephalus according to claim 15, wherein a distal end of the puncture member (4) is provided with a puncture tip having a cutting edge, the puncture tip is configured to puncture a dura mater to form a puncture opening in the dura mater, and the puncture tip is a circular or flat thin blade.

17. The system for treating hydrocephalus according to claim 16, wherein a puncture protective sheath is provided outside the puncture tip; and/or
the distal end of the puncture member (4) is provided with a radiopaque marker.

18. The system for treating hydrocephalus according to claim 15, wherein a distal anchoring member is connected to a distal end of the guide member (5), and/or a proximal deflection member is connected to a proximal end of the guide member (5).

19. The system for treating hydrocephalus according to claim 15, wherein the delivery system further comprises a dilation member configured to dilate the puncture opening; and/or
the delivery system comprises at least one radiopaque marker; and/or
the delivery system is connected to an operation handle, and the operation handle is configured to operate the delivery system to deliver the shunt.
